(19) Europäisches Patentamt
European Patent Office
Office européen des brevets

(11) Numéro de publication: 0 262 426 B1

(12) **FASCICULE DE BREVET EUROPEEN**

(45) Date de publication de fascicule du brevet: **04.12.91**

(51) Int. Cl.⁵: **C07C 47/42**, C07C 47/45, C07C 45/54, C07C 45/51

(21) Numéro de dépôt: **87112711.4**

(22) Date de dépôt: **01.09.87**

(54) **Procédé pour la préparation d'aldéhydes cycloaliphatiques.**

(30) Priorité: **25.09.86 CH 3847/86**

(43) Date de publication de la demande: **06.04.88 Bulletin 88/14**

(45) Mention de la délivrance du brevet: **04.12.91 Bulletin 91/49**

(84) Etats contractants désignés: **CH DE FR GB LI NL**

(56) Documents cités:

**TETRAHEDRON LETTERS, no. 29, 1963, pages 2087-2090, Pergamon press Ltd, GB; L.I. ZAKHARKIN et al.: "The preparation of aldehydes by reduction of esters of carboxylic acids with sodium aluminium hydride"**

**J. ORG. CHEM., vol. 41, no. 19, 1976, pages 3092-3096; D.R. DIMMEL et al.: "Allyl alcohol to saturated ketone isomerizations in the presence of alkali metal or n-butyllithium"**

(73) Titulaire: **FIRMENICH SA**
**Case postale 239**
**CH-1211 Genève 8(CH)**

(72) Inventeur: **Fehr, Charles, Dr.**
**6, chemin Ravoux**
**CH-1290 Versoix(CH)**
Inventeur: **Galindo, José**
**c/o FIRMENICH SA Case Postale239**
**CH-1211 Geneve 8(CH)**

(74) Mandataire: **Salvadori, Giuseppe, Dr.**
**c/o Firmenich S.A. Case Postale 239**
**CH-1211 Genève 8(CH)**

**EP 0 262 426 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a trait au domaine de la synthèse organique et, en particulier, elle concerne un procédé pour la préparation d'aldéhydes cycloaliphatiques à partir des esters d'alkyle correspondants.

Compte tenu de l'importance des composés aldéhydiques dans des domaines variés, soit en tant que produits intermédiaires de synthèse, soit en qualité de produits finaux utiles, notamment en tant qu'ingrédients de parfumerie et d'arômes, ce type de procédé revêt une grande importance industrielle. Il suffit ici de relever le rôle de certains aldéhydes cycloaliphatiques insaturés tels l'alpha- ou le bêta-cyclocitral ou le bêta-safranal. Ces composés sont généralement obtenus par des procédés complexes, d'où l'intérêt de disposer de nouvelles méthodes dont l'application serait à la fois simple et économique.

La présente invention apporte précisément une solution originale au problème posé par leur synthèse.

Elle a en effet pour objet un procédé pour la préparation d'aldéhydes cycloaliphatiques de formule

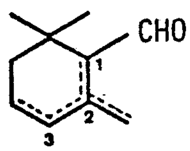

(I)

contenant soit une double liaison isolée dans la position 1 ou 2 (endocyclique ou exocyclique), soit deux doubles liaisons conjuguées dans les positions 1 et 3 ou 2 (exocyclique) et 3 du cycle, telles qu'indiquées par les pointillés, lequel procédé est caractérisé en ce qu'on effectue successivement les étapes réactionnelles suivantes :

a. déprotonation avec une base forte d'un ester de formule

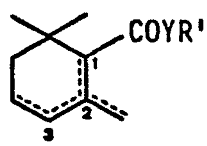

(II)

dans laquelle les lignes pointillées ont le sens indiqué plus haut, le symbole Y sert à désigner un atome d'oxygène ou de soufre et R' représente un reste alkyle linéaire ou ramifié, de préférence $C_1$-$C_6$, ou phényle, substitué ou non, la base forte étant constituée par le propyl- ou le butyl-lithium, ou le diméthyl-, diéthyl- ou le diisopropylamidure de lithium,

b. addition à l'énolate ainsi formé de formule

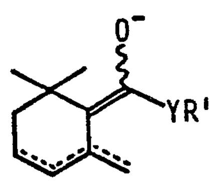

(III)

d'un agent réducteur pouvant fournir un anion $H^{\ominus}$ dans les conditions de la réaction, constitué par un hydrure de lithium trialkoxyaluminium ou l'hydrure de sodium bis(2-méthoxyéthoxy)aluminium, addition suivie, le cas échéant, du traitement du produit obtenu avec un halogénure de trialkyl-silyle, et

c. hydrolyse du produit résultant.

Le procédé décrit ci-dessus peut être représenté par le schéma réactionnel suivant :

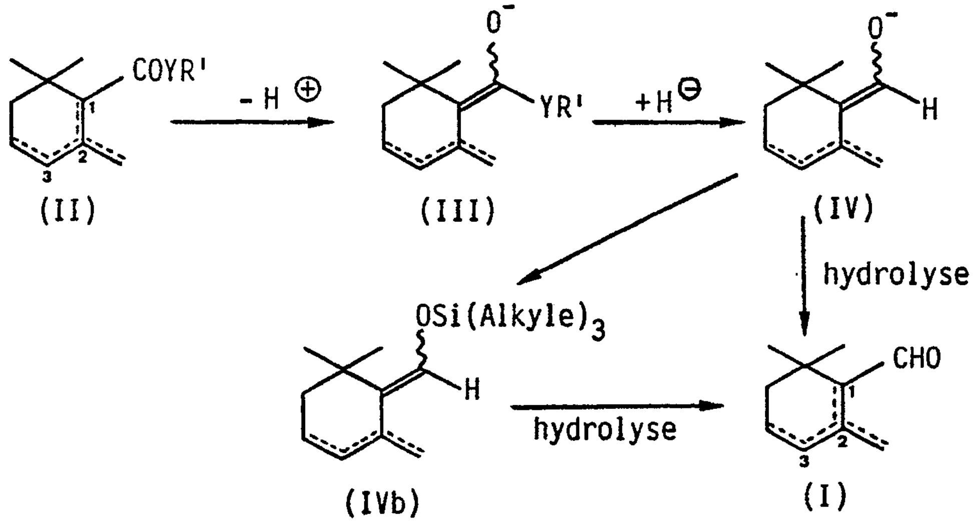


(II) (III) (IV) (IVb) (I)

A titre de produit de départ de formule (II), on peut utiliser un ester cycloaliphatique choisi parmi les esters alkyles inférieurs de l'acide alpha-, bêta- ou gamma-cyclogéranique ou de l'acide bêta- ou gamma-safranique. Parmi les esters préférentiels, il convient de mentionner l'alpha-, le bêta- et le gamma-cyclogéraniate de méthyle ou d'éthyle ainsi que leurs homologues supérieurs n-propyle, iso-propyle et butyle, ou le bêta- et gamma-safranate de méthyle ou d'éthyle. Il s'agit de produits disponibles commercialement ou qui peuvent être aisément accessibles par voie de synthèse selon des procédés connus. Comme produits de départ, on peut également utiliser des thioesters. A titre d'exemple, on peut citer parmi les thioesters préférentiels l'alpha- ou le bêta-thiocyclogéraniate de S-phényle.

La première étape du procédé, qui consiste en la déprotonation desdits esters de départ, s'effectue au moyen d'une base forte. Comme base forte, on peut utiliser un dérivé alkyle d'un métal alcalin, tel le propyl- ou le butyl-lithium, ou encore un amidure de métal alcalin, de préférence un amidure de lithium. C'est ainsi que des bases telles le diméthyl-, le diéthyl- ou le diisopropyl-amidure de lithium, constituent des bases préférentielles.

D'autres bases fortes telles le dicyclohexyl-amidure de lithium, ou celles représentées par les formules

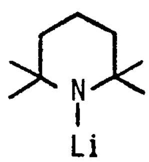 et $LiN[Si(CH_3)_3]_2$

peuvent également convenir.

Des mélanges constitués par deux des bases précitées peuvent également être employés.

La réaction s'effectue dans des solvants organiques inertes, par exemple en dissolvant l'ester de départ dans un éther, tel le tétrahydrofuranne, et la base dans un hydrocarbure aliphatique, cycloaliphatique ou aromatique, tels par exemple l'hexane, le cyclohexane, le benzène ou le toluène.

Sans vouloir préjuger de l'exactitude de l'interprétation mécanistique de la réaction qui caractérise cette première étape de la réaction, on peut affirmer que le produit formé se présente sous forme d'une entité transitoire dans les conditions de la réaction. Plusieurs indices indirects tendent toutefois à en confirmer la structure. Il nous a été par exemple possible d'isoler le dérivé silylé de formule

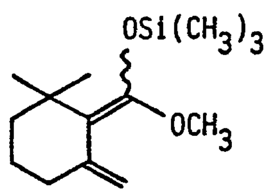

par adjonction du chlorure de triméthyl-silyle au mélange réactionnel, après traitement du gamma- ou bêta-cyclogéraniate de méthyle avec une base forte selon l'invention.

Ce fait suggère que l'entité transitoire se présente en bonne probabilité sous forme d'un énolate, tel qu'indiqué par la formule (IIIc), lequel pourrait trouver son origine soit dans la déprotonation directe de l'ester (II), soit dans l'action de la base forte utilisée (ou d'un nucléophile) sur un cétène-acétal de formule (IIIb) suivant le schéma que voici :

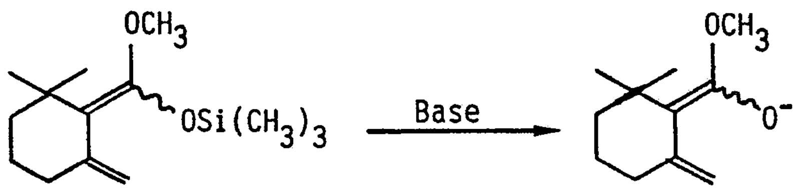


(IIIb) (IIIc)

Quant à la température de réaction de cette première étape du procédé de l'invention, elle peut se situer aux environs de la température ambiante ou être légèrement inférieure à celle-ci, par exemple à environ 15-25° C. Selon un mode d'exécution particulier du procédé de l'invention, en utilisant du n-butyl-lithium comme base et du bêta-cyclogéraniate de méthyle comme ester de départ, on peut opérer à environ 15° C.

Des températures inférieures peuvent également être employées. Par exemple, le traitement du 2,6,6-triméthyl-1-cyclohex-1-ène-1-carbothioate de S-phényle par du butyl-lithium s'effectue aisément à la température de -78° C.

A titre d'agent réducteur pouvant fournir un anion $H^{\ominus}$ dans les conditions de la réaction, on peut employer des hydrures métalliques, par exemple un hydrure de métal alcalin ou des hydrures complexes d'aluminium ou de bore tel l'hydrure de sodium bis(2-méthoxyéthoxy)aluminium (Vitride), le diéthyldihydroaluminate de sodium (OMH-1), l'hydrure de diisobutylaluminium (DIBAH), l'aluminohydrure de lithium ($LiAlH_4$), le borhydrure de lithium-triisobutyle, le borhydrure de lithium-triisoamyle ou d'autres complexes analogues connus sous le nom de Selectrides (marque enregistrée de Aldrich). Parmi les agents réducteurs préférentiels, il convient donc de mentionner ceux qui obéissent à la formule générale suivante :

$M^1 M^2 X_{4-n} H_n$

dans laquelle $M^1$ désigne un atome de bore ou d'aluminium, $M^2$ un atome d'un métal alcalin, de préférence le sodium et le lithium, et l'indice n représente un nombre entier dont la valeur est égale à 1, 2 ou 3.

La dernière étape s'effectue par hydrolyse du produit obtenu par traitement de celui-ci avec de l'eau, de préférence en milieu légèrement acide, par exemple au moyen d'une solution aqueuse glacée de chlorure d'ammonium. Elle peut également s'effectuer au moyen d'un dérivé hydroxylé, par exemple au moyen d'un alcool aliphatique. Le 2-butanol est utilisé de préférence.

Comme indiqué plus haut, l'hydrolyse qui caractérise l'étape c. du procédé peut s'effectuer directement sur le produit (IV), résultant de l'addition à l'énolate (III) de l'agent réducteur. Alternativement, on peut transformer le produit résultant en un éther silyle de formule

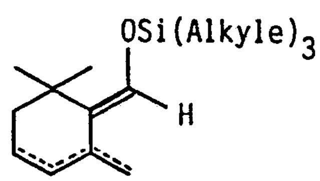

(IVb)

par addition d'un chlorure de trialkyl-silyle audit produit résultant (IV) et hydrolyser l'éther (IVb) pour fournir les aldéhydes désirés. Ceux-ci se présentent toutefois sous forme d'un mélange isomérique quelque peu différent de celui obtenu par hydrolyse "directe". Lorsqu'on soumet, par exemple, le bêta-safranate de méthyle au procédé de l'invention par traitement avec du butyl-lithium, suivi de réduction et hydrolyse avec une solution aqueuse concentrée de $NH_4Cl$, on obtient le safranal sous forme d'un mélange gamma-/bêta- dont le contenu isomérique est respectivement de 55/45. En effectuant la transformation préalable en l'éther

silanique (IVb), l'hydrolyse de ce dernier fournit par contre du bêta-safranal dont la pureté est d'environ 95%.

Les traitements usuels de séparation des phases, neutralisation et distillation permettent l'obtention des produits désirés.

L'invention est illustrée de manière plus détaillée par les exemples suivants dans lesquels les températures sont indiquées en degrés centigrades et les abréviations ont le sens usuel.

Exemple 1

Préparation d'alpha-cyclocitral

9,3 ml d'une solution 1,42 N de butyl-lithium dans l'hexane (0,0132 M) sont ajoutés goutte à goutte à -78° à une solution de 2,0 g (0,011 M) d'alpha-cyclogéraniate de méthyle dans 40 ml de tétrahydrofuranne (THF) anhydre.

Une fois l'addition terminée, on laisse remonter la température à +15° et additionne d'un trait à cette température 3,17 ml (0,011 M) d'une solution 70% de Vitride [hydrure de sodium bis(2-méthoxyéthoxy)-aluminium] dans le toluène. Le mélange réactionnel est chauffé à 40° pendant 45 min, puis on l'hydrolyse en le versant sur une solution aqueuse saturée glacée de chlorure d'ammonium. On extrait à l'éther, sépare la phase organique et la soumet aux traitements usuels pour fournir un résidu qui, par distillation, fournit 1,27 g d'alpha-cyclocitral ayant Eb. 60-65° /66 Pa (pureté 88% ; rend. 65%).

Exemple 2

Préparation de bêta-cyclocitral

163 ml d'une solution 1,42 N (0,231 M) de butyl-lithium dans l'hexane sont ajoutés rapidement à -10/+15° à une solution de bêta-cyclogéraniate de méthyle (30 g ; 0,165 M) dans 150 ml de THF. On laisse réagir 5 min à 15°, puis additionne d'un trait la solution obtenue à une suspension de 3,15 g (0,083 M) de $LiAlH_4$ dans 150 ml de THF. La température monte progressivement et se stabilise à 30°. On chauffe le mélange jusqu'à ce qu'il atteigne 40° et le maintient à cette température pendant 2 h, puis on le refroidit à environ -15° et additionne 44,85 g de chlorure de triméthyl-silyle (0,413 M). On laisse remonter à température ambiante, puis hydrolyse sous atmosphère d'azote en versant le mélange sur une solution aqueuse saturée glacée de chlorure d'ammonium.

On extrait à l'éther, soumet les extraits organiques aux traitements usuels et dissout le résidu, obtenu après évaporation, dans du THF. La solution ainsi formée est hydrolysée par traitement avec 0,5 g d'acide p-toluène-sulfonique pendant 20 min. On verse ensuite sur une solution aqueuse saturée de $NaHCO_3$/glace et extrait à l'éther.

La phase éthérée est ensuite soumise aux traitements usuels de séchage et concentration avant distillation. On obtient ainsi 16,9 g de bêta-cyclocitral (pureté 94% ; rend. 63%).

Exemple 3

Préparation de bêta-cyclocitral

54,2 ml (0,077 M) d'une solution 1,42 N de butyl-lithium dans l'hexane sont ajoutés à -10/+15° à une solution de 10,0 g (0,055 M) de bêta-cyclogéraniate de méthyle dans 150 ml de THF anhydre. Après avoir laissé le mélange à 15° pendant 5 min, on y additionne d'un trait 15,9 ml d'une solution à 70% de Vitride dans le toluène (0,055 M). La température du mélange est ensuite portée à 40° et à cette température on laisse réagir le mélange pendant 45 min.

Le mélange réactionnel est ensuite rapidement versé à 15° à une solution de 20,35 g (0,275 M) de 2-butanol dans 100 ml de THF. Dès la fin de la réaction, on ajoute rapidement 150 ml d'HCl à 5% dans l'eau puis on extrait à l'éther. Les extraits organiques combinés sont lavés, séchés sur $Na_2SO_4$ et concentrés pour fournir un résidu qui, par distillation dans un four à boules, livre 7,74 g de bêta-cyclocitral (pureté 76% ; rend. 70%). En effectuant l'hydrolyse au moyen d'une solution aqueuse saturée de chlorure d'ammonium (ou avec du HCl aqueux à la place de 2-butanol, on obtient un mélange bêta-/gamma-cyclocitral dont le rapport respectif des isomères est environ 70/30.

Exemple 4

Préparation de bêta-cyclocitral

2,0 g (0,011 M) de bêta-cyclogéraniate de méthyle sont ajoutés lentement à -78° à une solution de diisopropylamidure de lithium (1,05 équiv.) dans 30 ml de THF. Dès la fin de l'introduction, on laisse remonter la température et à 15° on ajoute d'un trait 3,17 ml (0,011 M) d'une solution à 70% de Vitride dans le toluène. On monte la température à 40° et, après 45 min, on traite le mélange réactionnel avec 4,07 g de 2-butanol (0,055 M). Après traitement suivant l'exemple précédent, on obtient par distillation à 45-50°/1,33 Pa, 1,31 g de bêta-cyclocitral (rend. 67%). En effectuant l'hydrolyse au moyen d'une solution aqueuse saturée de chlorure d'ammonium (ou avec du HCl aqueux) à la place de 2-butanol, on obtient un mélange bêta-/gamma-cyclocitral dont le rapport respectif des isomères est environ 70/30.
Comme l'alpha-cyclocitral s'isomérise facilement en bêta-cyclocitral, ce dernier peut aussi être synthétisé à partir d'un mélange d'alpha- et de bêta-cyclogéraniate de méthyle ou à partir de l'alpha seulement.

Exemple 5

Préparation de bêta-safranal

82,1 ml (0,117 M) d'une solution 1,42 N de butyl-lithium dans l'hexane sont ajoutés à -78° à une solution de 20,0 g (0,111 M) de bêta-safranate de méthyle dans 200 ml de THF.
Dès la fin de l'introduction, on laisse remonter la température du mélange à 15° et on le verse d'un trait à une suspension de 2,11 g (0,056 M) de $LiAlH_4$ dans 200 ml de THF.
On monte la température à 40° et laisse réagir environ 1 1/2 h. En effectuant l'hydrolyse à ce stade des opérations au moyen d'une solution aqueuse saturée de chlorure d'ammonium et en procédant comme indiqué aux exemples précédents, on obtient un mélange de bêta-/gamma-safranal dont le contenu isomérique respectif est de 45/55. On refroidit à -15° et additionne rapidement 30,14 g (0,278 M) de chlorure de triméthyl-silyle, puis la température est portée à valeur ambiante. On hydrolyse avec une solution à 5% d'HCl/glace, filtre et évapore. Par distillation du résidu, on obtient à 60-65°/66 Pa, 14,2 g de bêta-safranal (pureté 95% ; rend. 81%).

Exemple 6

Préparation de bêta-safranal

41,4 ml (0,059 M) d'une solution 1,42 N de butyl-lithium dans l'hexane sont ajoutés à -78° à une solution de 10,0 g (0,056 M) de bêta-safranate de méthyle. Dès la fin de l'introduction, on laisse remonter la température à 15°, puis additionne d'un trait 16,16 ml (0,056 M) d'une solution à 70% de Vitride dans le toluène. On monte la température à 40° et, après avoir laissé le mélange sous agitation pendant 45 min, on le traite avec 15,2 g (0,140 M) de chlorure de triméthyl-silyle. Après les traitements usuels, on obtient par distillation à 60-65°/66 Pa, 7,54 g de bêta-safranal (rend. 85%).

## Revendications

1. Procédé pour la préparation d'aldéhydes cycloaliphatiques de formule

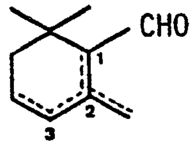

(I)

contenant soit une double liaison isolée dans la position 1 ou 2 (endocyclique ou exocyclique), soit deux doubles liaisons conjuguées dans les positions 1 et 3 ou 2 (exocyclique) et 3 du cycle, telles qu'indiquées par les pointillés, lequel procédé est caractérisé en ce qu'on effectue successivement les étapes réactionnelles suivantes :

a. déprotonation avec une base forte d'un ester de formule

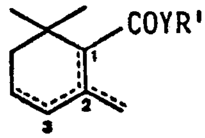

(II)

dans laquelle les lignes pointillées ont le sens indiqué plus haut, le symbole Y sert à désigner un atome d'oxygène ou de soufre et R' représente un reste alkyle linéaire ou ramifié, de préférence $C_1$-$C_6$, ou phényle, substitué ou non,

la base forte étant constituée par le propyl- ou le butyl-lithium, ou le diméthyl-, diéthyl- ou le diisopropylamidure de lithium,

b. addition à l'énolate ainsi formé de formule

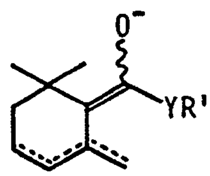

(III)

d'un agent réducteur pouvant fournir un anion $H^{\ominus}$ dans les conditions de la réaction, constitué par un hydrure de lithium trialkoxyaluminium ou l'hydrure de sodium bis(2-méthoxyéthoxy)aluminium, addition suivie, le cas échéant, du traitement du produit obtenu avec un halogénure de trialkyl-silyle, et

c. hydrolyse du produit résultant.

**2.** Procédé suivant la revendication 1. a., caractérisé en ce qu'on utilise la base à raison d'une quantité supérieure à la quantité équivalente de l'ester de départ (II).

**3.** Procédé selon la revendication 1, caractérisé en ce qu'on utilise comme ester de départ de formule (II) l'alpha- ou le bêta-cyclogéraniate de méthyle ou d'éthyle ou le bêta-safranate de méthyle ou d'éthyle, comme base forte le butyl-lithium ou le diisopropylamidure de lithium et comme agent réducteur pouvant fournir un anion $H^{\ominus}$ l'hydrure de sodium bis(2-méthoxyéthoxy)aluminium pour fournir, après hydrolyse, l'alpha- ou le bêta-cyclocitral et le bêta-safranal, respectivement.

## Claims

**1.** Process for the preparation of cycloaliphatic aldehydes of formula

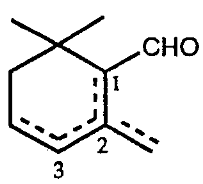

(I)

having either an isolated double bond in position 1 or 2 (endocyclic or exocyclic) or two conjugated double bonds in positions 1 and 3 or 2(exocyclic) and 3 of the ring as indicated by the dotted lines, which process is characterized in that the following reaction steps are carried out successively:

a. deprotonation with a strong base of an ester of formula

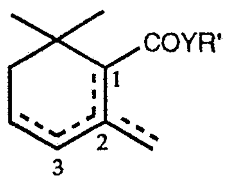

(II)

wherein the dotted lines have the above given meaning, symbol Y designates an oxygen or a sulphur atom and R' represents a linear or branched alkyl radical, preferably a $C_1$ to $C_6$ radical, or a substituted or unsubstituted phenyl, said strong base consisting of propyl- or butyllithium, or lithium dimethyl-, diethyl- or diisopropylamide;

b. addition to the thus formed enolate of formula

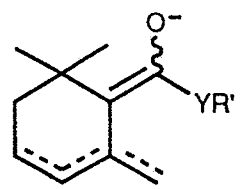

(III)

of a reducing agent capable of generating $H^{\ominus}$ anions in the reaction conditions, consisting of a lithium trialkoxyaluminium hydride or sodium bis(2-methoxyethoxy)aluminium hydride, addition optionally followed by the treatment of the obtained product with a trialkyl-silyl halide; and

c. hydrolysis of the resulting product.

**2.** Process according to claim 1.a., characterized in that said base is used in greater amount than the equivalent amount of starting ester (II).

**3.** Process according to claim 1, characterized in that the starting ester of formula (II) is methyl or ethyl $\alpha$- or $\beta$-cyclogeranate, or methyl or ethyl $\beta$-safranate, the strong base is butyl-lithium or lithium diisopropylamide and the reducing agent capable of generating $H^{\ominus}$ anions is sodium bis(2-methoxyethoxy)aluminium hydride to give, after hydrolysis, $\alpha$-or $\beta$-cyclocitral and $\beta$-safranal, respectively.

## Patentansprüche

**1.** Verfahren zur Herstellung von cycloaliphatischen Aldehyden der Formel

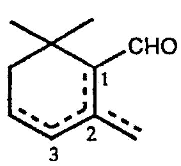

(I)

die entweder, wie durch die gestrichelten Linien angezeigt, eine alleinstehende Doppelbindung in der 1 oder 2 Ringstellung (endocyclischen oder exocyclischen), oder zwei conjugierten Doppelbindungen in den 1 und 3 oder 2(exocyclischen) und 3 Ringstellungen enthält, dadurch gekennzeichnet, daß man die folgenden Reaktionsstufen schrittweise durchführt:

a. Deprotonierung eines Esters der Formel

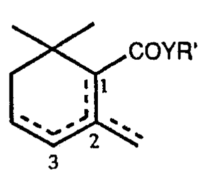

(II)

worin die gestrichelten Linien die angebene Bedeutung haben, das Symbol Y ein Sauerstoffatom oder ein Schwefelatom bedeutet und R' für einen linearen oder verzweigten, vorzugsweise $C_1$-$C_6$ Alkylrest, oder für einen substituierten oder unsubstituierten Phenylrest steht, mit einer starken Base, die aus Propyl- oder Butyllithium, oder Lithium Dimethyl-, Diethyl- oder Diisopropylamid besteht,

b. Addierung eines Reduktionsmittel das unter den Reaktionsbedingungen ein $H^{\ominus}$ Anion bilden kann und aus einem Lithiumtrialkoxyaluminiumhydrid oder aus einem Natrium bis(2-methoxyethoxy) aluminiumhydrid besteht, an das so erhaltene Enolat der Formel

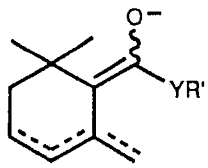

(III)

und, gebenenfalls, nach der Addierung, Behandlung des so erhaltenes Produkt mit einem Trialkylsilylhalogenid; und

c. Hydrolyse des erhaltenen Produktes.

**2.** Verfahren nach Anspruch 1.a., dadurch gekennzeichnet, daß man die Base in einer größeren Menge als das Äquivalent des Ausgangsesters (II) verwendet.

**3.** Verfahren nach Anspruch 1, dadurch gekennzeichnet, daß man als Ausgangsester der Formel (II), Methyl oder Äthyl $\alpha$- oder $\beta$-Cyclogeraniat, oder Methyl oder Äthyl $\beta$-Safranat, als starke Base Butyllithium oder Lithium Diisopropylamid und als Reduktionsmittel, das $H^{\ominus}$ Anionen entwickeln kann, Natrium bis(2-methoxyethoxy)aluminium-hydrid verwendet, um nach Hydrolyse beziehungsweise $\alpha$- oder $\beta$-Cyclocitral oder $\beta$-Safranal zu erhalten.